(19) Europäisches Patentamt
European Patent Office
Office européen des brevets

(11) Veröffentlichungsnummer: **0 197 266 B1**

(12) **EUROPÄISCHE PATENTSCHRIFT**

(45) Veröffentlichungstag der Patentschrift: **24.07.91**

(51) Int. Cl.$^5$: **C12Q 1/68**, G01N 33/52

(21) Anmeldenummer: **86102108.7**

(22) Anmeldetag: **19.02.86**

# (54) Verfahren und Träger zur Sequenzanalyse von Nucleinsäuren.

(30) Priorität: **26.02.85 DE 3506703**

(43) Veröffentlichungstag der Anmeldung:
**15.10.86 Patentblatt 86/42**

(45) Bekanntmachung des Hinweises auf die Patenterteilung:
**24.07.91 Patentblatt 91/30**

(84) Benannte Vertragsstaaten:
**CH FR GB IT LI SE**

(56) Entgegenhaltungen:
**EP-A- 0 067 921**
**EP-A- 0 070 687**
**EP-A- 0 092 688**
**US-A- 4 054 646**
**US-A- 4 129 781**

**SIEMENS RESEARCH & DEVELOPMENT REPORTS, Band 9, Nr. 4, 1980, Seiten 236-241, Berlin; B. FLAMME "Determination of layer thickness and refractive index of the layers of multilayer systems by ellipsometry"**

(73) Patentinhaber: **SAGAX Instrument AB**
**Nytorpsvägen 14**
**S-183 53 Täby(SE)**

(72) Erfinder: **Nygren, Hakan**
**Valbergsvägen 4 C**
**S-427 00 Billdal(SE)**
Erfinder: **Stenberg, Manne**
**Hedelundsvägen 5**
**S-417 43 Göteborg(SE)**

(74) Vertreter: **Haft, Berngruber, Czybulka**
**Postfach 14 02 46 Hans-Sachs-Strasse 5**
**W-8000 München 5(DE)**

EP 0 197 266 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Desoxyribonucleinsäure (DNA) ist ein doppelsträngiges Molekül mit zwei komplementären Ketten aus den vier Nucleotidbasen Adenin, Cytosin, Guanin und Thymin. Die beiden Stränge der DNA sind über Wasserstoffbrückenverbindungen miteinander verknüpft, wobei sich bestimmte Basenpaare, nämlich Adenin-Thymin und Cytosin-Guanin zusammenfinden. Die der DNA verwandte RNA enthält Ribose statt Desoxyribose und Uracil- statt Thyminresten. Die spezielle DNA-Verknüpfung zu zwei komplementären Ketten führt zu dem genetischen DNA-Code, welcher die genetische Information in allen lebenden Zellen, insbesondere der Chromosomen, darstellt. Bekannte Sequenzen von DNA oder RNA können in der Biotechnologie zur Darstellung der Sequenzen von unbekanntem genetischen Material oder zu Diagnosezwecken zur Identifizierung genetischen Materials aus Viren und Bakterien verwendet werden. Herkömmliche Verfahren zur Bestimmung von DNA- oder RNA-Hybridisierung beruhen auf der Bindung von radioaktiv oder enzymatisch markierten DNA- oder RNA-Sequenzen an komplementärem, einsträngigem DNA oder RNA und nachfolgender Erfassung der Markierung. Als feste Phasen werden in den meisten Fällen Cellulose oder andere Polysaccharide zur Immobilisierung von DNA verwendet und wirken als Rezeptoren für die markierte DNA-Sonde.

Hierzu ist es beispielsweise bekannt, (E. M. Southern, J. Mol. Biol., 98, 503, 1975), Fragmente der DNA durch Elektrophorese in Agarosegel zu trennen und die getrennten Fragmente zu einzelnen Strängen zu denaturieren, welche durch Diffusion auf Nitrocelluloseteile übertragen werden. Die getrennten Sequenzen werden dann identifiziert durch Inkubation radioaktiv markierter komplementärer Sequenzen, welche an ihre Targets gebunden werden. Das radioaktive Isotop wird dann lokalisiert durch Aktivierung einer photographischen Emulsion. Ferner ist bekannt (B.E. Noyes und G.R. Stark, Cell 5, 301, 1975), zum gleichen Zweck die denaturierte DNA bzw. RNA kovalent an Cellulose mittels einer Diazobenzylgruppe zu binden.

Sequenzen von DNA aus infektösen Mikroorganismen können als Sonden verwendet werden zur Erfassung von eventuell vorhandener komplementärer DNA in Proben von Serum, Mark, Fäkalstoffen oder dgl. zu Diagnosezwecken. Bislang werden hierzu radioaktiv oder enzymatisch markierte Sonden, die bekannte Nucleinsäuresequenzen aufweisen, verwendet.

Aus der EP-A-70 687 ist ein Verfahren zur Sequenzanalyse von DNA gemäß dem Oberbegriff des Patentanspruches 1 beschrieben, bei dem ein Nucleinsäurestrang einer Probe auf einem Träger immobilisiert wird. Mit diesem Nucleinsäurestrang werden komplemenäre Nucleinsäurestränge mit bekannter Sequenz in Kontakt gebracht, wobei sich komplementäre Sequenzen durch Hybridisierung vereinigen. Nach Abtrennen der nicht gebundenen Segmente werden die Markierungsstoffe aktiviert, so daß das dabei erzeugte Licht optisch wahrgenommen und zum Nachweis der Hybridisierung verwendet wird. Hierbei werden die Nucleinsäuresequenzen auf dem Träger z.B. mit Hilfe von aktivierten Glasoberflächen, Polyacrylamid, Agarose, Cellulose usw. immobilisiert, wobei diese Immobilisierung sämtlich durch Absorptionskräfte erfolgt.

In der europäischen Anmeldung EP-A-92 688 wird ein Verfahren und ein Träger zum Nachweis einer biochemischen Substanz bei einem immunologischen Verfahren beschrieben. Ein Antigen wird auf einem Träger aus Glas, Silizium oder Kunststoff immobilisiert, der eine aus einer Doppelschicht aus Siliziumoxid und Siliziumdioxid bestehende reflektierende Oberfläche aufweist, und mit einer flüssigen Probe in Berührung gebracht, die den korrespondierenden Antikörper enthält. Bei der Bindung des Antikörpers an das immobilisierte Antigen entsteht eine organische Schicht auf der Trägeroberfläche. Das Dickenwachstum dieser Schicht wird aus Interferenzänderungen bei der Reflexion von Licht an der reflektierenden Oberfläche bestimmt. Eine Verwendung dieses Verfahrens auf eine Sequenzanalyse geht aus dieser Druckschrift nicht hervor.

Aufgabe der Erfindung ist es, ein Verfahren zur Sequenzanalyse von Nucleinsäuren sowie einen hierfür verwendbaren Nachweisträger und ein Verfahren zur Herstellung dieses Trägers zu schaffen, bei welchem die Durchführung der Sequenzanalyse von Nucleinsäuren mit unmarkierten Sonden durchgeführt werden kann.

Diese Aufgabe wird gelöst durch die kennzeichnenden Merkmale der Ansprüche 1, 7 und 11, wobei in den Unteransprüchen Weiterbildungen der Erfindung gekennzeichnet sind.

Aus der DE-OS 23 30 702 ist ein Verfahren zum Nachweis spezifischer Proteine mit Hilfe eines an einen Träger gebundenen Antikörpers bekannt. Im Gegensatz dazu handelt es sich jedoch bei der Erfindung darum, daß eine zu untersuchende Nucleinsäure während der Analyse auf einem festen Träger immobilisiert wird, an den eine Nucleinsäure mit bekannter Sequenz chemisch gebunden ist und das Dickenwachstum der organischen Schicht gemessen wird.

Hierzu wird denaturierte einsträngige DNA bzw. RNA kovalent gebunden auf einer lichtreflektierenden Oberfläche des Trägers, und dieser Träger wird als Nachweismedium mit der zu untersuchenden Probe, welche denaturierte Nucleinsäure aufweist, deren Sequenz zu analysieren ist, in Berührung gebracht. Komplementäre denaturierte Nucleinsäure in der Probe wird an der am Träger vorhandenen, eine bestimmte Sequenz aufweisende einsträngige Nucleinsäure gebunden, wodurch durch optische Messung das Dickenwachstum der sich dabei bildenden organischen Schicht, durch welche insbesondere die Reflexionseigenschaften der reflektierenden Trägeroberfläche verändert werden, nachweisbar ist. Dieser Nachweis kann dadurch erfolgen, daß Änderungen der Lichtpolarisation nach der Reflexion an der Trägeroberfläche oder Änderungen der Interferenzfarben ermittelt werden. Zur Überwachung des Polarisationsverhaltens kann bevorzugt das in der europäischen Patentschrift 19 088 bzw. in der US-PS 43 32 476 beschriebene ellipsometrische Verfahren sowie die dort beschriebene ellipsometrische Vorrichtung zur Untersuchung der physikalischen Eigenschaften der Oberfläche einer Probe verwendet werden. Für den Nachweis der Änderung der Interferenzfarben des an der Trägeroberfläche reflektierten Lichts infolge der aufgewachsenen organischen Schicht eignet sich ein Trägerkörper, auf dessen reflektierender Oberfläche eine Doppelschicht, nämlich eine Reflexions-Antireflexionsschicht, vorhanden ist, auf welcher der Nucleinsäurestrang mit der bekannten Sequenz immobilisiert ist. Als derartiger Trägerkörper eignet sich insbesondere ein solcher, wie er in der DE-OS 32 15 484 beschrieben ist.

Ein geeigneter Trägerkörper, der bei der durchzuführenden Analyse mit der zu untersuchenden Probe als Nachweismedium in Berührung gebracht wird, besitzt bevorzugt eine Silicium- oder Aluminiumoberfläche, auf der ein Siliciumwasserstoff mit einer funktionellen Gruppe als Schicht aufgebracht ist. An dieser Silciumwasserstoffschicht wird dann der Nucleinsäurestrang mit der bekannten bzw. bestimmten Sequenz durch chemische Bindung, insbesondere kovalente Bindung, immobilisiert. In vorteilhafter Weise kann noch eine Zwischenschicht aus einem Polysaccharid, insbesondere Dextran, zur Immobilisierung der Nucleinsäure aufgebracht werden.

Von Vorteil ist bei der Erfindung, daß die Nucleinsäuresonden, welche die Nucleinsäure mit bekannter Eigenschaft, insbesondere Sequenz, enthalten, für den nachfolgenden Nachweis nicht mehr markiert werden müssen. Es können bei der Erfindung somit bekannte Proben mit unmarkierten Sonden untersucht werden. Der hierzu erforderliche Trägerkörper läßt sich einfach herstellen und besitzt einen einfachen Aufbau. Die Nachweisreaktion läßt sich in einfacher Weise in einem Vergleichsellipsometer, wie es beispielsweise im europäischen Patent 19 088 bzw. in der US-PS 43 32 476 beschrieben ist, durchführen. In dieser Vorrichtung lassen sich komplementäre Sequenzen, die sich an dem auf dem Träger befindlichen Nucleinstrang gebildet haben, visuell sichtbar machen und damit nachweisen und bestimmen. Der Träger wird dabei in der bekannten ellipsometrischen Vorrichtung als Probe eingesetzt. Man benötigt daher bei der Erfindung einen relativ geringen apparativen Aufbau im Gegensatz zu den bislang bekannten Verfahren.

Die reflektierende Trägeroberfläche besteht bevorzugt aus Siliciumdioxid oder Aluminiumoxid. Auf diese ist eine Schicht eines Siliciumwasserstoffs aufgebracht, der eine funktionelle Endgruppe enthält. Hierfür eignet sich insbesondere eine Amino-($NH_2$-)- oder eine

Epoxy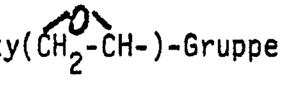-Gruppe.

Die Bindung der Siliciumwasserstoffschicht an der reflektierenden Oberfläche erfolgt spontan an Silanolgruppen bzw. Aluminiumhydroxylgruppen. Die denaturierte Nucleinsäure (DNA bzw. RNA) mit einer bestimmten bekannten Verknüpfungsfolge werden chemisch, insbesondere kovalent, über eine Diazobenzylgruppe direkt an die Siliciumwasserstoffschicht oder über eine Zwischenschicht aus einem Polysaccharid, beispielsweise Dextran, gebunden.

Die Probe wird vor der Analyse denaturiert, beispielsweise in 0,5 M Natriumhydroxid oder dgl. und anschließend neutralisiert, und nachfolgend wird noch ein Hybridisierungspuffer zugegeben, dessen Zusammensetzung eine langsame Verknüpfung der komplementären Nucleinsäurestränge, insbesondere der DNA- bzw. RNA-Stränge ermöglicht. Dieser Puffer kann beispielsweise aus 50 % Formamid, 0,05 M Natriumcitrat, 0,1 % Dodecylsulfat, 1 mM EDTA (Ethylendiamintetraessigsäure) bzw. deren Na-Salz, 0,02 % Albumin und 2 % Dextransulfat bestehen. Die Verknüpfung der komplementären Nucleinsäure aus der Probe mit dem kovalent am Träger immobilisierten Nucleinstrang äußert sich im Anwachsen der Dicke der dabei auf der Trägeroberfläche gebildeten organischen Schicht. Nach Spülen mit einer Kochsalzlösung und Wasser und anschließendem Trocknen läßt sich die Dicke der aufgewachsenen organische Schicht, wie schon erwähnt, mit Hilfe der Vergleichsellipsometrie (US-PS 43 32 476 oder EP 19 088) oder durch Messung der Änderung der Interferenzfarben von aus Siliciumoxiden bestehenden Doppelschichten an reflektierenden Trägerkörpern, wie sie beispielsweise in der DE-OS 32 15 484 beschrieben sind, ermitteln bzw. messen.

Im folgenden werden Ausführungsbeispiele zur Herstellung eines Trägers, welcher als Nachweismedium bei der Sequenzanalyse von Nucleinsäuren verwendet wird, im einzelnen beschrieben. Diese Träger besitzen eine lichtreflektierende Trägeroberfläche, auf welcher die denaturierte DNA oder

RNA durch kovalente Bindung aufgebracht ist.

Beispiel 1

Ein Siliciumplättchen oder Glasplättchen bzw. -scheibchen, auf welches Silicium oder Aluminium beispielsweise durch Aufdampfen aufgebracht ist, wird in einem Exsikkator angeordnet, der mit einem Einlaßhahn und einem Auslaßhahn versehen ist. Der Druck im Innern des Exsikkators wird auf etwa 10 mm Hg verringert und die Temperatur auf 80° C gehalten. Ein Kolben, der Epoxysiliciumwasserstoff enthält, wird an den Einlaß des Exsikkators angeschlossen, und der Einlaßhahn am Exsikkator wird geöffnet. Der Siliciumwasserstoff wird dabei auf der Oberfläche des mit Silicium bzw. Aluminium beschichteten Silicium- bzw. Glaskörpers durch Destillation aufgebracht, wobei innerhalb von etwa zwei Stunden eine epoxy-aktivierte Oberfläche entsteht. Die beschichteten Körper werden dann in eine Lösung von 12 % 2-Aminotiophenol in Aceton eingetaucht und über einen längeren Zeitraum, etwa 10 Stunden, in einem Inkubator aufbewahrt. Anschließend werden die beschichteten Körper gewaschen und bis zu ihrer weiteren Verwendung in Wasser aufbewahrt. Die beschichteten Körper werden dann in 1 M Salzsäure, welche 250 $\mu$g/ml Natriumnitrid aufweist, eine Stunde bei 4° C eingetaucht, gewaschen und in einer Feuchtekammer aufbewahrt. Isolierte reine DNA wird in 25 mM Phosphatpuffer gelöst, auf 90° C erhitzt, ein dem vierfachen Volumen der vorhandenen Lösung entsprechendes Volumen an Dimethylsulfoxid wird zugesetzt und im Zeitraum von etwa 10 Stunden tropfenweise auf die Schichtkörperoberfläche aufgebracht und gezüchtet. Die plättchenförmigen Schichtkörper werden dann wieder gewaschen und in einer Lösung von Albumin (10 $\mu$g/ml) in einem Phosphatpuffer durch Inkubation nachbehandelt, um die Oberfläche für unspezifische Adsorption zu inaktivieren.

Beispiel 2

Die Trägerplättchen werden mit Epoxysiliciumwasserstoff oder Aminosiliciumwasserstoff wie im Beispiel 1 behandelt. Anschließend wird Dextran an die epoxy- bzw. aminoaktivierte Oberfläche durch Inkubation in einer 20 %-Lösung von Dextran in Wasser gebunden. Hierdurch entsteht ein 20 bis 40 Å dicker Überzug aus Dextran auf der Oberfläche. Alternativ hierzu kann Dextran durch Inkubation mit 0,01 % Natriumperjodat bei pH 8,0, welches in einer Sephadex®-G25-Säule entsalzt ist, auf der mit Aminosiliciumwasserstoff beschichteten Oberfläche während etwa 10 Stunden gezüchtet werden. Das gebundene Dextran wird dann stabilisiert durch Inkubation mit einer 0,1 %igen Natriumborhydridlösung in 0,1 M Acetatpuffer bei pH 5,0. Anschließend wird 0,1 M Natriumhydroxid (10 ml) und 1,4 Butandioldiglycidylether (1 ml) auf die plattenförmigen Trägerkörper im Zeitraum von etwa 10 Stunden aufgebracht, und anschließend wird ebenfalls in einem Zeitraum von etwa 10 Stunden 12 % 2-Aminothiophenol in Aceton den Trägerplättchen zugegeben. Die Trägerplättchen werden dann in Wasser aufbewahrt. Isolierte und reine DNA oder RNA wird dann nach einer Nitridbehandlung der Platten wie im Beispiel 1 auf die Trägerplättchen aufgebracht.

Beispiel 3

Die Trägerplättchen werden mit einer 20 bis 40 Å dicken Schicht aus Dextran, wie im Beispiel 2 beschrieben, beschichtet. Die mit Dextran beschichteten Trägerplättchen werden dann zur Inkubation mit einer Lösung aus 0,2 g Natriumacetat in Wasser (10 ml), welches auch 1 g 1-((m-Nitrobenzyloxy)methyl)pyridiniumchlorid behandelt, anschließend in einem Ofen bei 70° C getrocknet und dann bei 140° C während etwa einer Stunde gebacken. Die Trägerplättchen werden dann zur Inkubation in einer 20 %igen Lösung von Natriumdithionid behandelt und mit Wasser gewaschen. Ferner werden dann die Trägerplättchen in 1 M Salzsäure, welche 250 $\mu$g/ml Natriumnitrid aufweist, etwa eine Stunde lang bei 4° C eingetaucht, gewaschen und in einer Befeuchtungskammer aufbewahrt. Abschließend wird dann isolierte, reine DNA bzw. RNA gelöst und durch Züchtung, wie im Beispiel 1 beschrieben, auf die vorstehend behandelten Trägerplättchen aufgebracht.

Im folgenden werden anhand der beiliegenden Figuren Ausführungsbeispiele für bei der Sequenzanalyse von Nucleinsäure verwendbaren Trägern beschrieben.

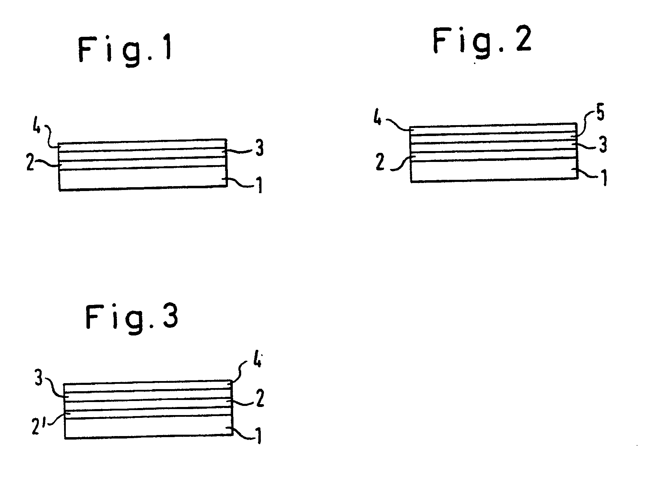

Bei den in den Figuren 1 bis 3 dargestellten Ausführungsbeispielen handelt es sich um schematische Darstellungen der in Form von Plättchen ausgebildeten Träger, wobei lediglich die Reihenfolge der Anordnung der einzelnen Schichten auf dem Grundkörper veranschaulicht werden soll, ohne daß die Verhältnisse der Schichtdicken der dargestellten Schichtdicken den in der Praxis vorhandenen Maßstäben entsprechen.

In der Fig. 1 befindet sich auf einem Trägergrundkörper 1, der beispielsweise aus Silicium oder Glas bestehen kann, eine Siliciumdioxidschicht 2, welche auch eine Aluminiumoxidschicht sein kann, die als reflektierende Oberfläche wirkt. Auf dieser Schicht befindet sich eine Schicht 3 aus Siliciumwasserstoff mit funktionellen Epoxy- oder Aminogruppen. Darüber befindet sich die Schicht 4, in welcher die auf dem Träger immobilisierte bzw. fixierte einsträngige Nucleinsäure, beispielsweise DNA, vorhanden ist.

Das Ausführungsbeispiel des in der Fig. 2 dargestellten Trägers entspricht im wesentlichen dem in der Fig. 1 und unterscheidet sich demgegenüber lediglich dadurch, daß zwischen der Siliciumwasserstoffschicht 3 und der Schicht 4 mit der fixierten denaturierten einsträngigen Nucleinsäure eine Polysaccharidschicht 5, insbesondere aus Dextran, angeordnet ist.

Bei dem in der Fig. 3 dargestellten Ausführungsbeispiel ist die reflektierende Oberfläche zweischichtig ausgebildet, wobei die auf dem Grundkörper 1 aufliegende erste Schicht 2' aus Siliciummonoxid und die zweite Schicht 2 aus Siliciumdioxid besteht. Die Wirkungsweise dieser Doppelschicht ist im einzelnen in der DE-OS 32 15 484 beschrieben.

**Patentansprüche**

1. Verfahren zur Sequenzanalyse von Nucleinsäure, insbesondere der Dexoxyribonucleinsäure (DNA) oder Ribonucleinsäure (RNA), bei dem eine zu untersuchende flüssige Probe, die denaturisierte Nucleinsäure aufweist, mit der lichtreflektierenden Oberfläche eines Trägers, an den ein Nucleinsäurestrang mit bestimmter Sequenz chemisch gebunden wurde, in Berührung gebracht und die Hybridisierung komplementärer Sequenzen der in der Probe vorhandenen denaturisierten Nucleinsäure mit dem an der Trägeroberfläche vorhandenen Nucleinsäurestrang optisch bestimmt wird, dadurch gekennzeichnet, daß der Nucleinsäurestrang mit bestimmter Sequenz mit einer kovalenten Bindung an den Träger (1) gebunden wird, und daß das Dickenwachstum der sich durch die Hybridisierung auf der Trägeroberfläche bildenden organischen Schicht optisch bestimmt wird.

2. Verfahren nach Anspruch 1, dadurch gekennzeichnet, daß das Dickenwachstum aus den Änderungen der Reflexionseigenschaften der reflektierenden Trägeroberfläche bestimmt wird.

3. Verfahren nach Anspruch 2, dadurch gekennzeichnet, daß das Dickenwachstum durch ellipsometrische Messung bestimmt wird.

4. Verfahren nach Anspruch 2, dadurch gekennzeichnet, daß das Dickenwachstum durch Interferenzmessung bestimmt wird.

5. Verfahren nach Anspruch 3, dadurch gekennzeichnet, daß das Dickenwachstum durch Vergleichsellipsometriemessung bestimmt wird.

6. Verfahren nach Anspruch 4, dadurch gekennzeichnet, daß das Dickenwachstum aus Interferenzänderungen bei der Reflexion von Licht an einer reflektierenden Oberfläche, die eine aus Siliciumoxid und Siliciumdioxid bestehende Doppelschicht aufweist, bstimmt wird.

7. Träger zur Durchführung des Verfahrens nach einem der vorhergehenden Ansprüche, für die Sequenzanalyse von Nucleinsäure, insbesondere der Desoxyribonucleinsäure (DNA) oder Ribonucleinsäure (RNA), wobei auf der Oberfläche des Trägers ein Nucleinsäurestrang mit bestimmter Sequenz chemisch gebunden ist, welcher mit einer zu untersuchenden flüssigen Probe, die denaturierte Nucleinsäure aufweist, in Berührung gebracht wird, wobei sich komplementäre Sequenzen der in der Probe vorhandenen denaturierten Nucleinsäure mit dem an der Trägeroberfläche vorhandenen Nucleinsäurestrang durch Hybridisierung vereinigen, dadurch gekennzeichnet, daß der eine bestimmte Sequenz aufweisende Nucleinsäurestrang durch kovalente Bindung an der lichtreflektierenden Trägeroberfläche haftet.

8. Träger nach Anspruch 7, dadurch gekennzeichnet, daß der Nucleinsäurestrang über eine Polysaccharidzwischenschicht (5) an der reflektierenden Trägeroberfläche kovalent gebunden ist.

9. Träger nach Anspruch 7 oder 8, dadurch gekennzeichnet, daß der Trägerkörper (1) an seiner lichtreflektierenden Oberfläche eine Siliciumdioxid- oder Aluminiumoxidschicht (2) aufweist, auf der eine Schicht (3) aus funktionellem Siliciumwasserstoff sich befindet, an welchem der Nucleinsäurestrang kovalent gebunden ist.

10. Träger nach Anspruch 8, dadurch gekennzeichnet, daß der Siliciumwasserstoff als funktionelle Gruppen Amino- oder Epoxygruppen aufweist.

11. Verfahren zur Herstellung eines Trägers nach Anspruch 7, dadurch gekennzeichnet, daß ein eine funktionelle Gruppe aufweisender Siliciumwasserstoff auf die reflektierende Oberfläche des Trägers (1) aufdestilliert, auf diese aktivierte Siliciumwasserstoffschicht durch Züchtung aus einer wässrigen Lösung eine Polysaccharidschicht aufgebracht und anschließend darauf der Nucleinsäurestrang aus einer Pufferlösung, in welcher die reine Nucleinsäure gelöst ist, gezüchtet wird.

## Claims

1. A method of sequence analysis of nucleic acid, in particular deoxyribonucleic acid (DNA) or ribonucleic acid (RNA), wherein a liquid sample to be examined, which includes denaturized nucleic acid, is contacted with the light reflecting surface of a support, to which a nucleic acid strand having a particular sequence is chemically bound and wherein the hybridization of complementary sequences of the denaturized nucleic acid present in the sample and the nucleic acid strand present at the support surface is determined optically, characterized in that the nucleic acid strand having the particular sequence is bound to the support (1) by covalent bonding, and that the increase of thickness of the organic layer formed by the hybridization on the support surface is determined optically.

2. A method according to claim 1, characterized in that the increase of thickness is determined from the change of the reflection properties of the reflecting support surface.

3. A method according to claim 1, characterized in that the increase of thickness is determined by ellipsometric measurement.

4. A method according to claim 2, characterized in that the increase of thickness is determined by interference measurement.

5. A method according to claim 3, characterized in that the increase of thickness is determined by comparative ellipsometric measurement.

6. A method according to claim 4, characterized in that the increase of thickness is determined from changes of interference during the reflection of light at a reflecting surface having a double layer consisting of silicon oxide and silicon dioxide.

7. A support for use in performing the method of sequence analysis of nucleic acid, particular desoxyribonucleic acid (DNA) or ribonucleic acid (RNA) according to one of the preceeding claims, wherein a nucleic acid strand having a particular sequence is chemically bound to the surface of the support and is contacted with a liquid sample to be examined, which includes denaturized nucleic acid, whereby complementary sequences of the denaturized nucleic acid present in .the sample unite by hybridization with the nucleic acid strand present at the support surface, characterized in that the nucleic acid strand having the particular sequence adheres to the light-reflecting support surface by covalent bonding.

8. A support according to claim 7, characterized in that the nucleic acid strand is covalently bound to the reflecting support surface through a polysaccharide intermediate layer (5).

9. A support according to claim 7 or 8, characterized in that the support body (1) has on its light-reflecting surface a silicon dioxide or aluminium oxide layer (2), on which a layer (3) of functional silicon hydride is located, to which the nucleic acid strand is covalently bound.

10. A support according to claim 9, characterized in that the silicon hydride has amino or epoxy groups as functional groups.

11. A method for producing a support according to claim 7, characterized in that a silicon hydride having a functional group is distilled onto the reflecting surface of the support (1), that a polysaccharide layer is applied from an aqueous solution onto the activated silicon hydride layer by incubation and that subsequently the nucleic acid strand is incubated thereupon from a buffer solution in which the pure nucleic acid is dissolved.

## Revendications

1. Un procédé pour analyser les séquences des acides nucléiques, acide désoxyribonucléique (ADN) ou acide ribonucléique (ARN) en particulier, dans lequel un échantillon liquide à examiner, qui contient de l'acide nucléique dénaturisé, est mis en contact avec la surface réfléchissant la lumière d'un support en liaison chimique avec une colonne d'acide nucléique à séquence déterminée et où l'hybridation de séquences complémentaires de l'acide nucléique dénaturisé contenu dans l'échantillon avec la colonne d'acide nucléique présente à la surface du support est déterminée par mesure optique, caractérisé par le fait que la colonne d'acide nucléique à séquence déterminée est liée au support (1) par une liaison covalente et que l'épaississement de la couche organique se formant à la surface du support en raison de l'hybridation est déterminé par mesure optique.

2. Un procédé selon la revendication 1, caractérisé par le fait que l'épaississement est déterminé à partir des modifications des propriétés de réflexion de la surface du support réfléchissant.

**3.** Un procédé selon la revendication 2, caractérisé par le fait que l'épaississement est déterminé par mesure ellipsométrique.

**4.** Un procédé selon la revendication 2, caractérisé par le fait que l'épaississement est déterminé par mesure d'interférence.

**5.** Un procédé selon la revendication 3, caractérisé par le fait que l'épaississement est déterminé par mesure ellipsométrique comparative.

**6.** Un procédé selon la revendication 4, caractérisé par le fait que l'épaississement est déterminé à partir des modifications d'interférence dans la réflexion de la lumière sur une surface réfléchissante à double couche constituée d'oxyde de silicium et de bioxyde de silicium.

**7.** Un support pour la mise en oeuvre du procédé selon l'une des revendications précédentes, procédé d'analyse des séquences des acides nucléiques, acide désoxyribonucléique (ADN) ou acide ribonucléique (ARN) en particulier, dans lequel une colonne d'acide nucléique à séquence déterminée est liée par liaison chimique à la surface du support qui est mis en contact avec un échantillon liquide à examiner contenant de l'acide nucléique dénaturé, des séquences complémentaires de l'acide nucléique dénaturé contenu dans l'échantillon s'unissant alors par hybridation à la colonne d'acide nucléique présente sur la surface du support, caractérisé par le fait que la colonne d'acide nucléique à séquence déterminée est fixée à la surface réfléchissant la lumière du support par liaison covalente.

**8.** Un support selon la revendication 7, caractérisé par le fait que la liaison covalente entre la colonne d'acide nucléique et la surface réfléchissante du support est établie par une couche intermédiaire de polysaccharide (5).

**9.** Un support selon la revendication 7 ou 8, caractérisé par le fait que le corps de support (1) présente, à sa surface réfléchissant la lumière, une couche de bioxyde de silicium ou d'oxyde d'aluminium (2) sur laquelle se trouve une couche (3) d'hydrogène silicié fonctionnel auquel la colonne d'acide nucléique est liée par liaison covalente.

**10.** Un support selon la revendication 8, caractérisé par le fait que l'hydrogène silicié présente des groupes amino ou epoxy comme groupes fonctionnels.

**11.** Un procédé de fabrication d'un support selon la revendication 7, caractérisé par le fait qu'un hydrogène silicié possédant un groupe fonctionnel est distillé sur la surface réfléchissante du support (1), que sur cette couche d'hydrogène silicié activée est appliquée une couche de polysaccharide, par culture, à partir d'une solution aqueuse, et que, enfin, la colonne d'acide nucléique est mise en culture dessus, à partir d'une solution tampon dans laquelle est dissous de l'acide nucléique pur.

Fig.1

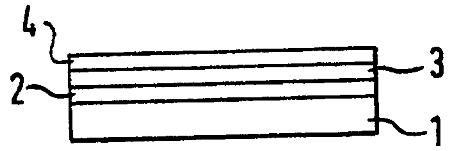


Fig.2

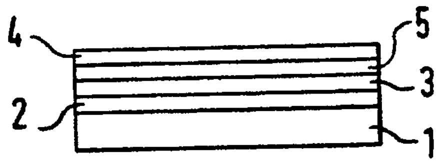


Fig.3